# EUROPEAN PATENT SPECIFICATION

(11) **EP 1 885 242 B1**
(45) Date of publication and mention of the grant of the patent: **20.10.2010**
(21) Application number: 06738431.3
(22) Date of filing: 02.03.2006
(51) Int. Cl.: A61B 5/05, A61N 1/18

(54) **INSTRUMENTS FOR NERVE MONITORING IN SPINAL SURGICAL PROCEDURES**
INSTRUMENTE ZUR NERVENÜBERWACHUNG WÄHREND CHIRURGISCHER BEHANDLUNGEN DES RÜCKENMARKS
INSTRUMENTS POUR LA SURVEILLANCE DU SYSTEME NERVEUX DANS LES PROCÉDURES DE CHIRURGIE SPINALE

(30) Priority: 04.03.2005 US 73265
(43) Date of publication of application: 13.02.2008
(73) Proprietor: Warsaw Orthopedic, Inc., Warsaw, IN 46581 (US)
(72) Inventor: MELKENT, Anthony, J., Memphis, Tennessee 38111 (US); POND, John, D., Germantown, Tennessee 38138 (US); BLACKWELL, Jonathan, E., Cordova, Tennessee 38016 (US); ADCOX, William, K., Memphis, Tennessee 38103 (US); FELTON, Sharonda, T., Cordova, Tennessee 38016 (US)
(74) Representative: Hutchinson, Glenn Stanley
(86) International application number: PCT/US2006/009368
(87) International publication number: WO 2006/094314

(56) References cited:
- US-A- 3 664 329
- US-A1- 2004 122 482

## Description

### BACKGROUND

Surgical devices for monitoring nerves have been employed to detect nerve proximity relative to the device. Such devices employ an electrical signal that is transmitted through the device to measure the nerve reaction based on the signal strength and nerve proximity. Such devices typically employ probes or needles with a distal end positionable in contact with the target tissue or and a proximal end manipulatable during the procedure to reposition the distal end if necessary. If bodily tissue, instruments or other structures impede access to the target tissue or implant during the surgical procedure, these structures must be repositioned or removed to prevent shunting of the electrical signal to these structures. US 2004/122482 discloses a device for proximity detection and confirmation of treatment of a target nerve as does US 5474558

There remains a need for surgical instruments and methods for nerve monitoring in spinal surgical procedures that reduce or eliminate the need for removal or repositioning of bodily tissue, surgical instruments, and other structures during the procedure while minimizing or eliminating the effect of shunting of the electrical signals to adjacent structures.

### SUMMARY

The present invention, which is defined by claim 1, generally relates to surgical instruments for use of the same, and more particularly, but not exclusively, relates to instruments for monitoring nerve proximity during spinal surgical problems.

The systems include an anchor engageable to a vertebra and an extender removably mounted to the anchor. The extender includes an insulating member to insulate adjacent tissue from electrical signals delivered to the anchor through the extender and to prevent shunting of the electrical signals to tissue, instruments, and other structures adjacent the extender. As the anchor is engaged in a vertebra with the extender mounted thereto, the proximity of the anchor to neural elements in the vertebra can be monitored by measuring the reaction of the neural elements to the electrical signals. The anchor can be repositioned or re-directed if necessary while maintaining the anchor and extender in removable engagement with one another.

These and other aspects will be discussed further below.

### BRIEF DESCRIPTION OF THE DRAWINGS

FIG. 1 is a perspective view of a nerve monitoring system including an anchor and an insulated extender extending from the anchor, and a rod extending through the anchor.
FIG. 2 is a section view along line 2-2 of Fig. 1.
FIG. 3 is an elevation view of another embodiment insulating member in an unassembled condition.
FIG. 4 is the insulating member of FIG. 3 in an assembled condition.
FIG. 5 is a right hand end view of the assembled insulating member of FIG. 4.
FIG. 6 is a perspective view of another embodiment insulated extender.
FIG. 7 is a section view along line 7-7 of FIG. 6.
FIG. 8 is an elevation view in partial section of another embodiment anchor with an insulated extender and a plate member adjacent the anchor.

### DESCRIPTION OF THE ILLUSTRATED EMBODIMENTS

For the purposes of promoting an understanding of the principles of the invention, reference will now be made to the embodiments illustrated in the drawings and specific language will be used to describe the same. It will nevertheless be understood that no limitation of the scope of the invention is thereby intended. Any such alterations and further modifications in the illustrated devices, and such further applications of the principles of the invention as illustrated herein are contemplated as would normally occur to one skilled in the art to which the invention relates.

The present invention is directed to systems for monitoring nerve proximity during spinal surgical procedures. In one form, the systems and methods include an anchor engageable to bony tissue of a vertebral body and an extender removably mounted to the anchor. The extender includes a body made from an electrically conductive material. The body can be electrically coupled to an electrical signal source for delivery of electrical signals through the body of the extender to the anchor removably coupled thereto. The proximity of neural elements relative to the anchor can be monitored as the anchor is engaged to the vertebral body. The extender includes an insulating member extending about the body that insulates the body of the extender and prevents the electrical signal from being shunted to adjacent bodily tissue, instruments and other structures adjacent the extender.

The extender can be any type of structure removable mountable to the anchor. The extender may provide a passage through bodily tissue to the anchor mounted to the distal end thereof when the anchor is positioned in the patient. The passage may be enclosed by the extender, or the passage may be open along all or a portion of the length of the extender. The extender may further facilitate manipulation of the anchor in the body of the patient by, for example, maintaining engagement of the anchor as it is engaged to the vertebra and facilitating repositioning of a portion of the anchor in the patient in minimally invasive procedures. The extender may also allow application of forces to the anchor and/or to the bony tissue in which the anchor is engaged in order to, for example, reduce displacement between vertebrae, or compress or distract vertebrae.

In one embodiment, the insulating member of the extender is a coating on the body of the extender. In another embodiment, the insulating member includes at least a first portion and a second portion removably engageable to one another about the body of the extender. In a further embodiment, the insulating member is a sleeve positioned about the body of the extender. The sleeve can be removably or non-removably engage thereto. The insulating member can extend from a distal end of the extender to a location spaced distally of the proximal end of the extender. An electrical lead can extend from the proximal end of the extender where it can be electrically coupled to a source operable to generate electrical signals. The insulating member may be autoclavable for re-use, or may be disposable for one-time use.

In one embodiment, the extender defines a passage extending therealong in communication with the anchor removably engaged thereto. A driver instrument can be positioned through the passage and engaged to the anchor to drive the anchor into the vertebra while an electrical signal is delivered to the anchor through the extender. Since the insulating member insulates the electrical signal from tissue, instruments, and other structures adjacent the extender, the extender can be employed in a minimally invasive access portal in contact with the adjacent tissue or instruments during engagement of the anchor to the vertebra.

The anchors discussed herein can be multi-axial or uni-axial anchors. The anchors include a distal lower portion that is engageable in a vertebral body, and a proximal receiver to which an implant can be engaged. In one embodiment, the lower portion is in the form of a bone screw with a threaded shaft and a proximal head that is pivotally captured in the receiver. In another embodiment, the receiver is formed as one piece with the lower portion in a uni-axial arrangement. In other embodiments, the lower portion can be in the form of a hook, staple, cable, tether, suture anchor, interbody fusion implant, artificial disc implant, bolt, or other structure engageable to bony tissue. The receiver can be configured with a receptacle to receive a rod or other elongated linking member that extends between one or more additional anchors secured to one or more additional vertebrae. The receiver can also be provided in the form of an elongated stem, shaft or enlarged head about which a linking element is positioned, such as a spinal plate.

The extender can be coupled to the anchor in any manner allowing releasable attachment of the extender to the anchor. The extender can include internal or external threads for engaging corresponding ones of external or internal threads of the receiver of the anchor. The extender can include one or more components which clamp the receiver of the anchor. The extender can include one or more members extending along the body thereof that engage one or more receptacles in the receiver of the anchor. Extenders which snap fit, frictionally engage, provide an interference fit, or otherwise releasably engage the receiver of the anchor are contemplated. In still another embodiment, the extender includes one or more members that engage the lower portion of the anchor rather than or in addition to the receiver.

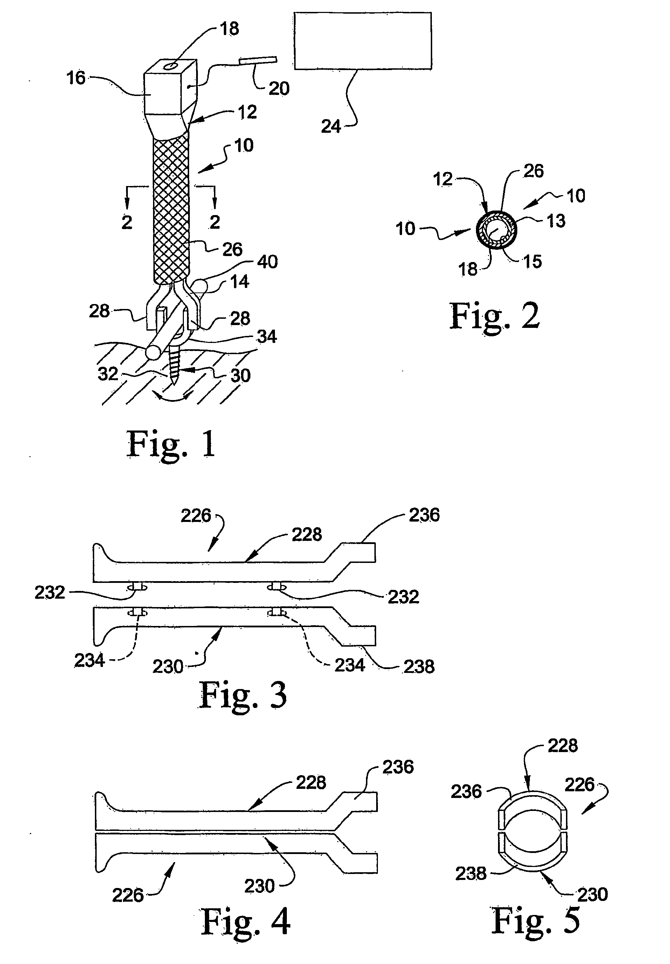

Referring now to Figs. 1 and 2, there is shown an extender 10 including a body 12 extending between a distal end 14 and a proximal end 16. An insulating member 26 extends about body 12 along at least a portion of the length of body 12. A passage 18 extends through body 12 and opens at distal and proximal ends 14, 16. Body 12 includes a lead 20 extending therefrom that is adjacent proximal end 16. Lead 20 can be electrically coupled to an electrical signal source 24. Signal source 24 is operable to generate at least an electrical signal deliverable through lead 20 to body 12. Signal source 24 may include controls, displays, memory, executable programs, or any other components or systems that may be desirable for generating electrical signals and measuring or indicating proximity to anchor 30. In one embodiment, signal source 24 is part of the NIM-Spine™ System marketed by Medtronic, Inc. System 10 also has applications with other nerve monitoring systems.

In another embodiment, extender 10 is not provided with lead 20. Rather, an instrument operable to deliver electrical signals, such as a probe or other instrument, is positioned in contact with a portion of body 12 not protected with insulating member 26. For example, the probe can be placed in contact with proximal end 16 and the electrical signal from the probe is delivered through body 12 to the anchor 30 coupled thereto.

Insulating member 26 can be in the form of a coating that is applied about all or a portion of the perimeter of body 12, or in the form of a sleeve that is fitted about all or a portion of the perimeter of body 12. In one embodiment, insulating member 26 is in the form of a sleeve that is flexible to tightly fit about body 12. In a further embodiment, insulating member 26 has a rigid body structure and is mounted about all or a portion of body 12. In another embodiment, insulating member 26 comprises two or more components that can be secured about all or a portion of body 12. In any embodiment, insulating member 26 may be comprised of any suitable electrically insulating material.

Anchor 30 is releasably engaged to the distal end 14 of extender 10. Anchor 30 includes a lower portion 32 engageable to bony tissue and a receiver 34 for engagement with an implant or other device. In one embodiment, lower portion 32 of anchor 30 is a threaded shaft to threadingly engaging bony tissue. The threaded shaft can be provided with self-drilling and/or self-tapping thread profiles to facilitate insertion into bony tissue. In another embodiment, the threaded shaft is configured for insertion in a pre-drilled and pre-tapped hole in the vertebral body.

In one embodiment, anchor 30 is a pedicle screw. Receiver 34 can be in the form of a U-shaped saddle having a receptacle to receive an implant positionable along the spinal column, such as rod 40. In one embodiment, the saddle is pivotal relative to lower portion 32, and lower portion 32 is pivotally captured in receiver 34. For example, lower portion 32 can be provided with an enlarged head at a proximal or upper end thereof that is pivotally captured in a bowl forming a distally opening receptacle in receiver 34. When lower portion 32 is engaged to the bony tissue, receiver 34 can be pivotally adjusted and repositioned as needed for engagement with rod 40. In another embodiment, the receiver 34 is integral with and formed as a single piece with the lower portion 32, providing a uni-axial anchor 30. Furthermore, a set screw, washer, crown, cap or other device may be provided for engagement within and/or about receiver 34 to secure rod 40 thereto.

In one procedure, extender 10 is releasably mounted to anchor 30, and the assembly can be inserted through a minimally invasive access portal for engagement of anchor 30 with bony tissue of a vertebra, such as the pedicle of the vertebra. The minimally invasive access portal can be provided by a micro-incision, a sleeve, a sleeve with an expandable working channel, a retractor blade, or two or more retractor blades of a retractor system.

The assembly can be guided to position anchor 30 in a desired trajectory or path into the vertebra using fluoroscopic imaging, endoscopic viewing, or other suitable viewing or imaging systems. Lower portion 32 can be engaged to the vertebra by positioning a driving instrument through passage 18 and into engagement with anchor 30. As lower portion 32 is advanced into engagement with the vertebra, the proximity of neural elements to lower portion 32 can be monitored. If the electrical signal delivered to lower portion 32 from body 12 comes within sufficient proximity of a neural structure, then reaction of the nerve to the electrical signal is noted and the engagement of lower portion 32 can be re-directed or stopped to prevent impingement upon the neural structure.

In one embodiment, extender 10 includes distal arms 28 movable toward and away from one another to selectively grip and release receiver 34 therebetween. As shown in Fig. 2, body 12 includes an outer sleeve 13 and an inner sleeve 15 received within the outer sleeve 13. Insulating member 26 extends about outer sleeve 13. Arms 28 comprise a portion of the distal end of the inner sleeve 15, and are movable toward and away from one another by moving outer sleeve 13 distally and proximally relative to the inner sleeve 15 to selectively grip and release anchor 30 from between arms 28. Examples of extender configurations including an inner sleeve and an outer sleeve are provided in U.S. Patent No. 6,530,929; U.S. Patent Application No. 10/769,569 filed on January 30, 2004; and U.S. Patent Application No. 10/674,036 filed on September 29, 2003.

In Figs. 3-5; there is shown another embodiment insulating member 226 that includes a first component 228 and a second component 230. First and second components 228, 230 are each configured to extend about a portion of the perimeter of body 12 of extender 10. Component 228, 230 are removably engageable with one another about body 12 of extender 10 to insulate body 12 and prevent shunting of electrical signals delivered through body 12 to bodily tissue, surgical instruments and other structures adjacent body 12 of extender 10.

In the illustrated embodiment, first and second component 228, 230 include outwardly extending feet 236, 238 that extend along and receive the outwardly extending arms 28 ofbody 12. Feet 236, 238 provide an insulating member that extends along the distal portion 14 of body 12 engaged to anchor 30 so that body 12 of extender 10 does not include any non-insulated portion positionable in the body of the patient

First and second components 228, 230 can include projections 232 and recesses 234. Projections 232 can be received in aligned ones of the recesses 234 to secure the first and second components 228, 230 to one another about body 12 of extender 10. Other coupling arrangements are also contemplated. For example, first and second components 228, 230 can be engaged about body 12 by clips, bands, adhesives, pins or other suitable coupling arrangements.

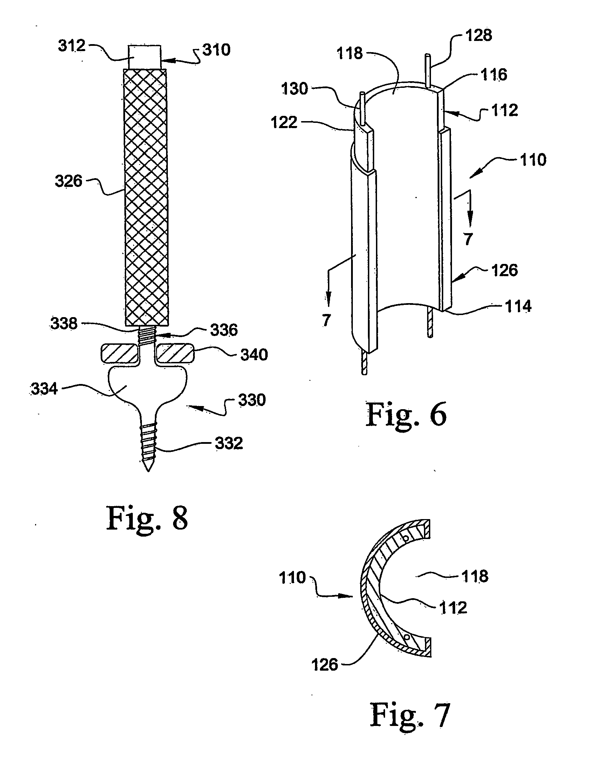

Figs. 6-7 show another embodiment of an extender 110 including is body 112 expending between a distal end 114 and a proximal end 116. An insulating member 126 extends at least partially about body 112. Body 112 is made from an electrically conductive material to deliver an electrical signal from a lead or probe in electrical communication with body 112 to an anchor coupled to distal end 114. Insulating member 126 is made from a material having sufficient insulative properties to insulate bodily tissue, surgical instruments, and other structures adjacent to body 112 from the electrical signal delivered through body 112.

In the embodiment of Figs. 6-7, body 112 includes a C-shaped cross-section defining passage 118 extending therethrough. Securing members 128,130 are provided through or along body 112 to releasably engage an anchor adjacent to distal end 114 and secure body 112 to the anchor. In the illustrated embodiment, securing members 128,130 are elongated pins that are rotatable relative to body 112 to threadingly engage their respective distal ends to threaded holes in the receiver of the anchor. Other mounting structures are also contemplated, including those that clamp, frictionally engage, or otherwise releasably mount extender 110 to the anchor.

Body 112 is positionable to retract bodily tissue along extender 110, and also to provide passageway 118 to the anchor or surgical site adjacent distal end 114 to facilitate engagement of the anchor to the vertebra and positioning of implants adjacent the anchor. Insulating member 126 can also include a C-shaped cross-section to match that of body 112. The C-shaped cross-section forms passage 118 to provide access to the anchor adjacent distal end 114.

Insulating member 126 can be in the form of a coating, sheath, separable components, or other structure that extends about at least a portion of the outer retracting surface of body 112 opposite passage 118. In the illustrated embodiment, insulating member 126 can be positioned longitudinally along body 112 for removable engagement therewith. The inner concave wall surface of body 112 defining passage 118 need not be covered by an insulating member since the outer retracting surface maintains the body tissue out of contact with the inner wall surface. In another embodiment, the insulating member 126 covers all surfaces of body 112.

Referring now to Fig. 8, there is provided another embodiment anchor 330 and insulated extender 310. Anchor 330 is engageable to bony tissue of a vertebral body, and may include a lower portion 332 having a shaft with a thread profile to threadingly engage a vertebra. Any other suitable bone engaging structure is also contemplated for lower portion 332 as discussed above with respect lower portion 32 of anchor 30. A receiver member 334 is provided at the proximal end of lower portion 332, and can be fixed or pivotal relative thereto. Receiver member 334 includes an upper portion 336 extending proximally therefrom. Upper portion may include any suitable structure for receiving an implant and for coupling the implant to anchor 330.

In the illustrated embodiment, upper portion 336 includes a removably mounted extender 310 extending from a mounting portion 338. Extender 310 includes an insulating member 326 extending thereabout to insulate adjacent bodily tissue and/or surgical instruments from an electric signal delivered through extender 310 to anchor 330. Insulating member may include any suitable configuration as discussed above. Anchor 330 is engageable to the vertebra with a driving tool engaged to receiver portion 334, by a driving tool engaged to anchor 330 about extender 310, or by a driving tool positioned through a passage (not shown) of body 312. The positioning of lower portion 332 and its proximity to neural elements is monitored by delivery of an electrical signal through extender 310 to lower portion 332. An implant 340, such as a plate, is positionable about extender 310 and movable therealong to facilitate positioning of implant 340 adjacent receiver portion 334. Further examples of such anchors and implant assemblies are provided in U.S. Patent Application Ser. No. 10/959,668, filed on October 5, 2004.

In one embodiment, extender 310 is integrally formed with lower mounting portion 338 of upper portion 336, and is removable upon application of sufficient torque to extender 310. Upper portion 336 can be provided with a break-off portion between extender 310 and mounting portion 338. Implant 340 is securable about lower mounting portion 338 with a set screw, nut or other securing device. Extender 310 can be removed before securing the implant 340, and can remain attached until the implant 340 is secured to mounting portion 338.

While the invention has been illustrated and described in detail in the drawings and foregoing description, the same is to be considered as illustrative and not restrictive in character, it being understood that only the preferred embodiment has been shown and described and that the scope of the invention is as defined by the following claims.

## Claims

1. A system for monitoring nerve proximity during a spinal surgical procedure in a patient, comprising:
an anchor (30) engageable to a vertebral body;
an extender (10) including an elongated body (12) extending between a distal end (14) removably mountable to said anchor and a proximal end (16), said body being comprised of an electrically conductive material and including a passage (18) that extends and opens at and between said distal and proximal ends thereof, said passage being sized for receipt of a surgical instrument engageable to said anchor to engage said anchor to the vertebra, the extender further including an insulating member (26) about at least a portion of said body of said extender, said insulating member being comprised of a material having properties to insulate structures adjacent to said portion of said body from an electrical signal delivered through said body of said extender to said anchor when said extender is mounted to aid anchor in the patient

2. The system of claim 1, wherein the passage is in communication with the anchor removably engaged to the extender.

3. The system of claim 1, wherein the insulating member extends from a distal end of the extender to a location spaced distally of the proximal end of the extender.

4. The system of claim 1, wherein said passage is enclosed by said body or wherein said passage is open along at least a portion of a length of said body.

5. The system of claim 1, wherein said anchor includes a lower portion (32) for engaging the vertebral body and a receiver (34) for engagement with an implant (40) positionable along the vertebral body when said lower portion is engaged to the vertebral body.

6. The system of claim 5, wherein the implant is selected from a group consisting of a rod and a plate.

## Patentansprüche

1. System zur Überwachung der Nervenproximität während einem Eingriff der Wirbelsäulenchirurgie in einem Patienten,wobei das System folgendes umfasst:
einen Anker (30), der mit einem Wirbelkörper eingreifen kann;
eine Verlängerungseinrichtung (10) mit einem elongierten Körper (12), die sich zwischeneinem diestalen Ende (14), das entfernbar an dem genannten Anker angebracht werden kann, und einem proximalen Ende (16) erstreckt, wobei der genannte Körper ein elektrisch leitfähiges Material umfasst und einen Durchgang (18) aufweist, der sich zwischen den genannten distalen und proximalen Enden erstreckt und zwischendiesen öffnet, wobei der genannte Durchgang für die Aufnahme eines chirurgischen Instruments bemessen ist, das mit dem genannten Anker eingreifen kann, um einen Eingriff des genannten Ankers mit dem Wirbel zu bewirken,wobei die Verlängerungseinrichtung ferner ein isolierendes Element (26) um mindestens ein Teilstückdes genannten Körpers der genannten Verlängerungseinrichtung aufweist, wobei das genannte isolierende Element ein Material mit Eigenschaften zum Isolieren von Strukturen angrenzend an das genannte Teilstück des genannten Körpers von einem elektrischen Signal umfasst, das durch den genannten Körper der genannten Verlängerungseinrichtung dem genannten Anker zugeführt wird, wenn die genannte Verlängerungseinrichtung an dem genannten Anker in dem Patientenangebracht wird.

2. System nach Anspruch 1, wobei sich der genannte Durchgang in Kommunikation mit dem Anker befindet, der entfernbar mit der Verlängerungseinrichtung eingreift.

3. System nach Anspruch 1, wobei sich das isolierende Element von einem distalen Ende der Verlängerungseinrichtung zu einer Position erstreckt, die distal zu dem proximalen Ende der Verlängerungseinrichtung mit Abstand angeordnet ist.

4. System nach Anspruch 1, wobei der genannte Durchgang durch den genannten Körper eingeschlossen wird, oder wobei der genannte Durchgang entlang zumindest einem Teilstück einer Länge des genannten Körpers offen ist.

5. System nach Anspruch 1, wobei der genannte Anker ein unteres Teilstück(32) zum Eingriff mit dem Wirbelkörper aufweist sowie eine Aufnahmeeinrichtung (34) zum Eingriff mit einem Implantat (40), das entlang des Wirbelkörpers positioniert werden kann, wenn das genannte untere Teilstückmit dem Wirbelkörper eingreift.

6. System nach Anspruch 5, wobei das Implantat aus einer Gruppe ausgewählt wird, die eine Stange und eine Platte umfasst.

## Revendications

1. Système pour surveiller la proximité des nerfs pendant une procédure chirurgicale spinale chez un patient, comprenant :
un ancrage (30) pouvant venir en prise avec un corps vertébral ;
un extenseur (10) comprenant un corps allongé (12) s'étendant entre une extrémité distale (14) montée de manière amovible sur ledit ancrage et une extrémité proximale (16), ledit corps étant composé d'un matériau électriquement conducteur et comprenant un passage (18) qui s'étend et s'ouvre au niveau de et entre lesdites extrémités distale et proximale de celui-ci, ledit passage étant dimensionné pour la réception d'un instrument chirurgical pouvant venir en prise avec ledit ancrage pour mettre en prise ledit ancrage avec la vertèbre, l'extenseur comprenant en outre un élément isolant (26) autour d'au moins une partie dudit corps dudit extenseur, ledit élément isolant étant composé d'un matériau ayant des propriétés pour isoler les structures adjacentes à ladite partie dudit corps d'un signal électrique transmis à travers ledit corps dudit extenseur vers ledit ancrage lorsque ledit extenseur est monté sur ledit ancrage dans le patient

2. Système selon la revendication 1, dans laquelle le passage est en communication avec l'ancrage mis en prise de manière amovible avec l'extenseur.

3. Système selon la revendication 1, dans lequel l'élément isolant s'étend d'une extrémité distale de l'extenseur vers un emplacement espacé de manière distale de l'extrémité proximale de l'extenseur.

4. Système selon la revendication 1, dans lequel ledit passage est fermé par ledit corps ou dans lequel ledit passage est ouvert le long d'au moins une partie d'une longueur dudit corps.

5. Système selon la revendication 1, dans lequel ledit ancrage comprend une partie inférieure (32) pour venir en prise avec le corps vertébral et un récepteur (34) pour venir en prise avec un implant (40) pouvant être positionné le long du corps vertébral lorsque ladite partie inférieure est en prise avec le corps vertébral.

6. Système selon la revendication 5, dans lequel l'implant est sélectionné parmi le groupe comprenant une tige et une plaque.
